# FASCICULE DE BREVET EUROPEEN

(11) **EP 3 952 941 B1**
(45) Date de publication et mention de la délivrance du brevet: **03.08.2022**
(21) Numéro de dépôt: 20713904.9
(22) Date de dépôt: 27.03.2020
(51) Int. Cl.: A61M 60/148, A61M 60/205, A61M 60/422

(54) **POMPE CARDIAQUE A COUPLAGE MAGNETIQUE ET A FLUX INVERSE**
HERZPUMPE MIT MAGNETISCHER KOPPLUNG UND RÜCKSTRÖMUNG
HEART PUMP WITH MAGNETIC COUPLING AND REVERSE FLOW

(30) Priorité: 10.04.2019 FR 1903836
(43) Date de publication de la demande: 16.02.2022
(73) Titulaire: Fineheart, 33600 Pessac (FR)
(72) Inventeur: HADDADI, Mohammad, 33700 Merignac (FR); GARRIGUE, Stéphane, Begles 33130 (FR); HADDADI, Maryam, 33700 Merignac (FR); BREDENBREUKER, Lars, 33600 Pessac (FR); MASCARELL, Arnaud, 37250 Montbazon (FR)
(74) Mandataire: IPAZ
(86) Numéro de dépôt international: PCT/EP2020/058869
(87) Numéro de publication internationale: WO 2020/207833

(56) Documents cités:
- US-A1- 2018 311 421

## Description

[La présente invention se rapporte à une pompe, notamment axiale, destinée à être immergée dans un fluide.

La présente concerne plus particulièrement mais pas uniquement une pompe pour assistance ventriculaire. Il s'agit par exemple d'une pompe alimentée par batterie et destinée à être insérée dans un corps humain pour aider à la circulation du sang.

L'insuffisance cardiaque (IC), incapacité progressive du cœur à fournir un débit sanguin nécessaire aux besoins métaboliques d'un individu dans la vie courante, est la seconde cause de mortalité dans les pays occidentaux. Le traitement de l'insuffisance cardiaque, qui consiste à augmenter le débit sanguin de façon adaptée aux besoins du patient, progresse mais reste encore insuffisant.

On connaît aussi le document US6234772 décrivant une pompe rotative implantable. Cette pompe est de type à entrainement magnétique et permet de forcer la circulation du sang en évitant toute zone stagnante. Ce document reste muet quant à une quelconque mise en place efficace de la pompe.

On connaît également le document EP2734251 B1 décrivant une pompe miniaturisée adaptée à une implantation cardiaque. Dans ce document l'accent est mis sur le fait que la circulation du sang à travers la pompe doit être à flux constante, sans zones de stase d'écoulement susceptibles de provoquer la formation de thromboses. Il est préconisé de laver les roulements avec un apport constant de sang frais car la chaleur et les contraintes géométriques de ces zones les rendent potentiellement prédisposés à la formation de thromboses.

Ces deux documents de l'art antérieur concernent une pompe cardiaque de type centrifuge et réalisant un bypass à l'extérieur du cœur.

La présente invention a pour but une pompe intraventriculaire qui aspire du sang à l'intérieur du cœur et rejette ce sang également à l'intérieur du cœur en direction des valves.

La présente invention a aussi pour but une pompe intraventriculaire dont le fonctionnement évite toute création de thromboses.

On atteint au moins l'un des objectifs avec une pompe destinée à être immergée dans un fluide, comprenant :
- un carter fixe doté d'une partie supérieure formant chambre de propulsion de fluide vers l'extrémité supérieure, et une partie inférieure formant stator et reliée de manière fixe à la partie supérieure,
- au moins une ouverture latérale entre la partie supérieure et la partie inférieure, et formant une chambre d'admission du fluide depuis l'extérieur vers la chambre de propulsion,
- un bloc moteur formé par :
   - ledit stator dans lequel sont disposés des premiers éléments magnétiques,
   - un rotor sous la forme d'une cloche coiffant au moins partiellement la tête du stator et comprenant des seconds éléments magnétiques destinés au couplage magnétique avec les premiers éléments magnétiques du stator, la cloche ayant au moins une ouverture sur sa partie supérieure de façon à créer un flux inverse du fluide depuis la chambre d'admission jusqu'à la base du stator via un passage entre le rotor et le stator,
- un arbre de transmission comprenant au moins un bras de liaison permettant de maintenir la cloche au-dessus du stator, l'axe de l'arbre de transmission étant superposé avec l'axe de rotation de la cloche,
- des pales latérales disposées entre la cloche et l'arbre de transmission et destinées à guider le flux inverse dans ledit passage entre le rotor et le stator.

La pompe selon l'invention est de préférence une pompe axiale. C'est-à-dire que le déplacement du fluide est parallèle à l'axe de rotation du rotor, la poussée est axiale.

Avec la pompe selon l'invention, on crée un flux inverse au flux principal destiné à traverser la pompe. Le circuit de circulation conventionnel du fluide démarre par une admission par la chambre d'admission, un passage dans le carter supérieur, puis une propulsion vers l'orifice de sortie supérieur de la quantité de fluide utile. La présente invention est notamment remarquable par le fait que son arrangement permet de créer un flux en sens inverse du flux principal. Ce flux inverse est éjecté vers la base du stator et joue ainsi un rôle de flux de nettoyage. En d'autres termes, par une disposition astucieuse d'ouverture dans la cloche, on utilise le mouvement de rotation prévu pour le flux principal, pour créer un flux inverse.

La cloche et le stator constituent avantageusement un moteur sans balais, ou « moteur brushless », à rotor externe. La partie mobile, qui est la cloche dans la présente invention, est donc située à l'extérieur du stator et est réliée à l'arbre de transmission qui comme on le verra plus loin sert de moyen de propulsion ou de turbine pour propulser le fluide vers la tête du carter puis vers l'extérieur.

De préférence, la liaison entre la cloche et l'arbre de transmission est rigide.

La pompe comprend des pales latérales disposées entre la cloche et l'arbre de transmission et destinées à guider le flux inverse dans le passage entre le rotor et le stator.

Ces pales latérales peuvent être orientées ou présenter une courbure. Une telle réalisation permet de guider efficacement le fluide dans le passage entre le rotor et le stator et créer ainsi le fluide inverse. Les dimensions et formes des pales peuvent être déterminées de façon à obtenir un flux continu ou pulsé, sans cavitation et laminaire. Avec la configuration du moteur, il est prévu d'atteindre des vitesses de 15 à 60 cm/s, en fonction de la vitesse de rotation des pales et donc du moteur. De préférence, le passage entre le rotor et le stator présente une largeur comprise entre 0.4 et 1mm, par exemple 0.6mm.

Selon une caractéristique avantageuse de l'invention, les bras de liaison constituent lesdites pales latérales. Ces pales latérales peuvent être disposées entre la paroi interne de la cloche et l'arbre de transmission, mais elles peuvent également être réalisées dans le prolongement de l'extrémité supérieure de la cloche, semblable à une cloche fermée à laquelle des ouvertures auraient été réalisées en laissant des arêtes de liaison. D'autres modes de réalisations peuvent être envisagés pour rendre solidaire la cloche et l'arbre de transmission en utilisant ou non directement les pales.

De préférence, l'ouverture est de forme circulaire tout autour de l'arbre de transmission.

Selon un mode de réalisation avantageux, la pompe selon l'invention peut être une pompe cardiaque intraventriculaire destinée à être fixée à l'apex d'un cœur, le passage entre le rotor et le stator débouchant directement dans la zone de rencontre entre la paroi interne du ventricule et la paroi externe du stator. Ainsi, le passage entre le rotor et le stator débouche dans la zone où le risque de thrombose est élevé. Le fluide inverse a pour rôle de créer une circulation du fluide dans cette zone de façon à éviter toute stagnation.

Par ailleurs, la pompe peut présenter des dimensions telles que la chambre d'admission se trouve à une distance de 1 à 3 cm de l'apex et l'orifice de sortie de la pompe se trouve à l'intérieur du ventricule à une distance de 1 à 3 cm en amont de la valve aortique. Une telle réalisation concerne le domaine des pompes cardiaques intégrées dans le ventricule, pompant et éjectant le sang dans le cœur dans la direction de la valve aortique.

Selon une caractéristique avantageuse de l'invention, en fonctionnement la cloche et l'arbre de transmission sont destinés à être en sustentation magnétique grâce aux éléments magnétiques, l'extrémité inférieure de l'arbre de transmission et l'extrémité supérieure du stator peuvent alors coopérer de façon à maintenir l'arbre de transmission dans son axe de révolution en phase de rotation.

Un système de bagues et roulements peut être utilisé pour le maintien de l'arbre de transmission, mais la présente invention utilise de préférence le maintien en sustentation par pivot.

L'extrémité supérieure du stator peut comporter une zone de pivot concave apte à recevoir l'extrémité inférieure convexe, dite pivot d'entrée, de l'arbre de transmission. Avec une telle réalisation, en fonctionnement, le fluide passe entre le pivot d'entrée et la zone de pivot de façon à éviter au maximum la thrombose en maintenant un rinçage permanent de cet interstice entre les pièces concave du stator et convexe du rotor.

En phase de repos, l'arbre de transmission peut se reposer sur le stator, dans ce cas le pivot d'entrée et la zone de pivot sont notamment en contact. Mais en fonctionnement, il existe un espacement entre le pivot d'entrée et la zone de pivot. Cet espacement permet à la fois la rotation de l'arbre de transmission et à la fois le maintien de cet arbre de transmission dans son axe de révolution.

Selon une variante, l'extrémité supérieure du stator peut comporter une zone de pivot convexe apte à recevoir l'extrémité inférieure concave, dite pivot d'entrée, de l'arbre de transmission. Le principe de fonctionnement reste identique au cas où le pivot d'entrée est convexe. D'autres arrangements peuvent être envisagés avec des formes plus ou moins complexes mais permettant à la fois la libre rotation et le maintien dans l'axe de révolution (ou de rotation) de l'arbre de transmission.

Selon un mode de réalisation avantageux de l'invention, l'arbre de transmission peut comporter ou être connecté à une partie supérieure dotée de pales, par exemple hélicoïdales, permettant une aspiration de fluide principal depuis la chambre d'admission vers la sortie de la pompe.

En complément notamment de ce qui précède, la pompe selon l'invention peut comprendre :
- à l'entrée de la partie supérieure du carter, un inducteur doté de pales de guidage pour rendre l'écoulement du fluide linéaire en direction de la partie supérieure de l'arbre de transmission ;
- la partie supérieure de l'arbre de transmission peut être un propulseur comprenant un corps central de forme évasée et destiné à créer de l'énergie cinétique;
- au moins une pale hélicoïdale réalisée autour dudit corps central; cette pale hélicoïdale ayant un profil extérieur évasé et comportant des spires à pas d'enroulement croissant et tendant vers l'infini ; le volume intérieur du carter étant complémentaire à la forme évasée de ladite au moins une pale hélicoïdale.

L'arbre de transmission entraîne donc une turbine ou un propulseur doté de pales pour aspirer efficacement le fluide depuis la chambre d'admission et l'éjecter en sortie de la pompe. De ce fait, la pompe joue son rôle de circulation du fluide dans le sens principal. En même temps, le fluide inverse permet de remuer le fluide présent dans la base du stator et éviter ainsi une stagnation, source d'éventuelles thromboses.

Selon un autre mode de réalisation de l'invention, la partie supérieure de l'arbre de transmission est un propulseur pouvant comprendre une tige dans l'axe centrale fixée à une turbine conçue pour effectuer des mouvements de rotation relativement au carter, cette turbine étant un cylindre creux d'axe de révolution confondu avec ladite tige et destiné au passage du flux principal du fluide, cette turbine comprenant au moins une pale interne disposée sur sa paroi interne et conçue pour faire circuler le fluide provenant de la chambre d'admission.

Selon un mode de réalisation avantageux de l'invention, la partie supérieure de l'arbre de transmission est un propulseur pouvant comprendre un corps destiné à créer de l'énergie cinétique, ce corps comprenant à son sommet un pivot de sortie apte à coopérer avec un guide fixé au carter, ce guide présentant une forme complémentaire à celle du pivot de sortie de façon à maintenir ce pivot de sortie stable en rotation et à former un espacement entre le pivot de sortie et le guide. Selon l'invention, ce guide comprend une ouverture centrale dans l'axe de révolution du pivot de sortie pour un passage de fluide depuis l'intérieur du carter vers l'extérieur via l'espacement et l'ouverture.

L'espacement peut présenter une largeur entre 20 et 80 µm. A titre d'exemple, on peut envisager un espacement de 50µm et une vitesse de fonctionnement du moteur à 4000 tr/min.

Avec la pompe selon l'invention, l'ouverture centrale dans le guide permet le passage du fluide et évite la stagnation de fluide dans le creux du guide lorsque celui-ci ne présente pas d'ouverture. Avec l'invention, on crée un interstice entre le pivot de sortie et le guide pour le passage de fluide lorsque la pompe est en fonctionnement. Avantageusement, un flux est ainsi maintenu dans cet interstice de sorte que le fluide, qui est notamment du sang, n'y stagne pas.

Cette disposition ne gêne en rien la fonction du guide qui est de maintenir la turbine dans son axe lors des rotations et dans le carter.

En fonctionnement, il existe donc un espacement entre le pivot de sortie et le guide. Cet espacement permet à la fois la rotation de l'arbre de transmission et à la fois le maintien de cet arbre de transmission dans son axe de révolution.

Selon l'invention, le pivot de sortie peut présenter une forme convexe et le guide peut alors comprendre une ouverture centrale dans l'axe de révolution du pivot de sortie pour le passage de fluide depuis l'intérieur du carter vers l'extérieur. Une telle réalisation permet le passage du fluide et évite la stagnation de fluide dans le creux du guide lorsque celui-ci ne présente pas d'ouverture.

De préférence, le pivot de sortie comprend une protubérance pénétrant dans l'ouverture du guide.

La protubérance permet de guider efficacement le passage du fluide.

Selon l'invention, le guide peut être constitué d'au moins une pale d'un redresseur (« straightener » en anglais) et/ou d'un diffuseur (« inducer » en anglais). En effet, la pompe peut être équipée d'un redresseur et/ou d'un diffuseur disposé à l'intérieur du carter au niveau du sommet de la turbine. On peut donc utiliser un redresseur seul, un diffuseur seul, un redresseur et un diffuseur ou un unique composant faisant office de redresseur et diffuseur. Le redresseur et/ou le diffuseur est fixé au carter et comporte des pales, qui pour une pompe cardiaque intraventriculaire, dirigent le fluide de sortie vers l'aorte.

Selon une caractéristique avantageuse de l'invention, le pivot de sortie peut être convexe de forme demi-sphérique, conique ou pyramidale.

Avantageusement, la pompe selon l'invention peut être une pompe cardiaque intraventriculaire destinée à être fixée à l'apex d'un ventricule gauche ou d'un ventricule droit ou d'un ventricule systémique.

D'autres avantages et caractéristiques de l'invention apparaîtront à l'examen de la description détaillée d'un mode de mise en œuvre nullement limitatif, et des dessins annexés, sur lesquels :
[Fig. 1] est une vue générale d'une pompe cardiaque intraventriculaire selon l'invention,
[Fig. 2] est une vue schématique d'une partie inférieure de la pompe selon l'invention,
[Fig. 3] est une vue schématique d'une turbine selon l'invention,
[Fig. 4] est une vue schématique d'une partie supérieure de la pompe comprenant un pivot de rotation de la turbine selon l'invention,
[Fig. 5] est une vue schématique très simplifiée d'un redresseur selon l'invention,
[Fig. 6] est une vue schématique d'une partie supérieure de la pompe comprenant illustrant un autre mode de réalisation d'un pivot de rotation de la turbine selon l'invention.

Les modes de réalisation qui seront décrits dans la suite ne sont nullement limitatifs; on pourra notamment mettre en œuvre des variantes de l'invention ne comprenant qu'une sélection de caractéristiques décrites par la suite isolées des autres caractéristiques décrites, si cette sélection de caractéristiques est suffisante pour conférer un avantage technique ou pour différencier l'invention par rapport à l'état de la technique antérieur. Cette sélection comprend au moins une caractéristique de préférence fonctionnelle sans détails structurels, ou avec seulement une partie des détails structurels si cette partie uniquement est suffisante pour conférer un avantage technique ou pour différencier l'invention par rapport à l'état de la technique antérieur.

En particulier toutes les variantes et tous les modes de réalisation décrits sont prévus pour être combinés entre eux dans toutes les combinaison où rien ne s'y oppose sur le plan technique.

Sur les figures, les éléments communs à plusieurs figures conservent la même référence.

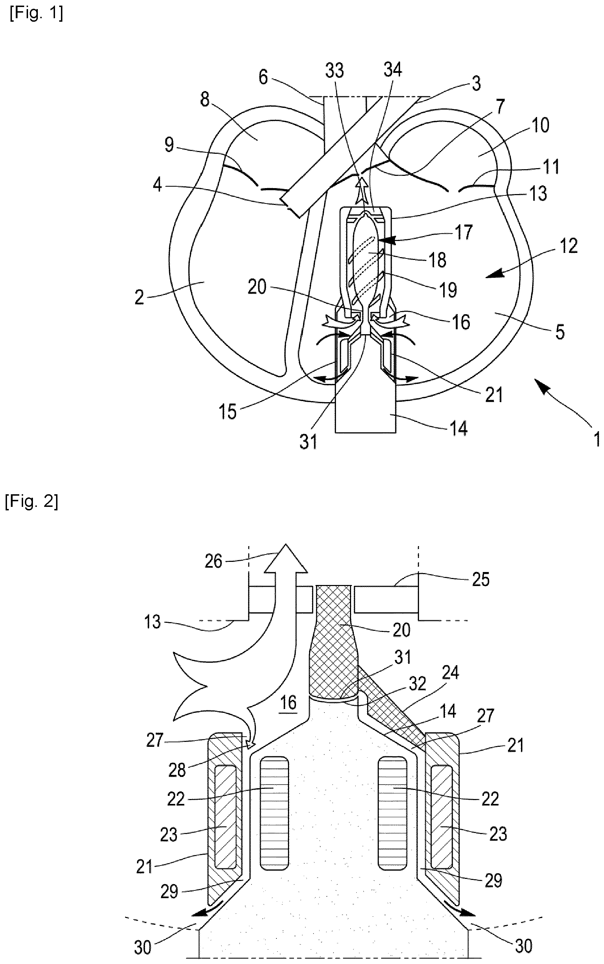

Sur la figure 1 est illustrée une pompe cardiaque intraventriculaire.

Le cœur est globalement désigné par la référence 1. On distingue le ventricule droit 2 qui a pour fonction d'éjecter le sang vers l'artère pulmonaire 3 à travers des valves sigmoïdes 4. Le ventricule gauche 5 a pour fonction de réaliser la circulation systémique en éjectant le sang rempli d'oxygène vers l'aorte 6 via des valves sigmoïdes 7.

L'oreillette droite 8 alimente le ventricule droit 2 en sang via des valves auriculo-pulmonaires 9. L'oreillette gauche 10 alimente le ventricule gauche 5 en sang via les valves auriculo-pulmonaires 11.

La pompe selon l'invention est globalement référencée en 12. Elle est fixée à l'apex du ventricule gauche 5. Elle peut être connectée de façon filaire ou sans fil à une unité de gestion (non représentée) externe au cœur. Elle peut être connectée à une o plusieurs sondes ou capteurs (non représentés) pour la détection du rythme cardiaque ou autre.

La partie inférieure de la pompe logeant le moteur peut être partiellement à l'extérieur du cœur, partiellement dans l'épaisseur de l'apex ou entièrement dans le cœur. Dans l'exemple de la figure 1, la partie inférieure de la pompe est partiellement à l'extérieure du cœur ce qui peut être un avantage pour la maintenance du moteur notamment.

La pompe comprend un carter composé d'une partie supérieure 13 reliée de façon rigide à une partie inférieure 14 au moyen d'éléments de liaison 15. Ces éléments de liaison peuvent comprendre une ou plusieurs tiges 15 reliant les deux parties 13 et 14 tout en laissant de larges passages pour le sang.

La partie inférieure 14 constitue le stator d'un moteur. Le carter est destiné à rester fixe. Entre la partie supérieure 13 et la partie inférieure 14, se trouve une chambre d'admission 16 qui est un espace ouvert, seulement entravé par les éléments de liaisons 15. En fonctionnement, la pompe est prévue pour aspirer le sang contenu dans le ventricule 5 via la chambre d'admission 16, le conduire à travers la partie supérieure 13 du carter et l'éjecter par la sortie supérieure vers la valve 7.

Pour aspirer le sang, la pompe comprend une turbine (« impeller » en anglais) 17 conçue à partir d'un corps oblongue 18 autour duquel est enroulée une ou plusieurs pales hélicoïdales 19. En rotation, la turbine aspire le sang et le propulse vers la sortie. Il s'agit de la fonction principale de la pompe. Le flux principal est donc celui qui est pompé par la turbine 17.

La turbine est portée par un arbre de transmission 20 qui comporte à l'extrémité opposée de la turbine une cloche 21. L'ensemble turbine 17, arbre de transmission 20 et cloche 21 est rigide et apte à effectuer des rotations. Pour ce faire, la cloche 21 constitue le rotor du moteur formé avec le stator fixe 14. Ce moteur 14 et 21 est un moteur de type « brushless » à rotor externe. Il s'agit d'une machine synchrone dotée d'un système électronique de commande (non représenté) accessible de l'extérieur du cœur ou non.

La turbine est apte à réaliser des mouvements de rotation selon son axe et relativement au carter 13, 14 qui reste fixe. Dans l'axe de rotation de la turbine se trouve un pivot d'entrée 31 et un pivot de sortie 33 qui maintiennent la turbine dans son axe lorsque celle-ci est en sustentation magnétique lors des mouvements de rotation. Dans une variante non représentée, l'une ou les deux zones de pivot peuvent comprendre des liaisons à roulements autorisant la rotation de la turbine.

Sur la figure 2, on distingue un peu plus en détail la chambre d'admission 16 et le moteur 14, 21. La chambre d'admission 16 est l'espace ouvert entre l'arbre de transmission 20, la cloche 21 et le carter supérieur 13.

Des enroulements statoriques 22 sont disposés dans le stator 14 à proximité d'aimants permanents 23 disposés dans la cloche 21, l'ensemble étant conçu en combinaison avec d'autres composants notamment électroniques (non représentés) pour constituer un moteur brushless.

L'arbre de transmission 20 est rigidement relié à la cloche 21 au moyen de pales latérales 24. L'activation du champ magnétique statorique par des moyens électroniques entraîne la rotation de la cloche 21, par conséquent la rotation de l'arbre de transmission 20 ainsi que de la turbine 17. Le fluide contenu dans le ventricule et tout autour de la pompe, pénètre par la chambre d'admission 16, continue dans la partie supérieure 13 du carter via un inducteur 25. Ce flux de fluide montant selon la représentation de la figure 2 est le flux principal 26. Selon l'invention, la cloche 21 présente une ouverture supérieure 27 à travers laquelle une partie du fluide est amenée à circuler. Il s'agit d'un flux inverse 28 descendant selon le schéma de la figure 2. Ce flux inverse est maintenu continu et laminaire grâce aux pales latérales 24 qui le guide vers un passage 29 présent entre la cloche 21 et le stator 14. Ce passage 29 amène le flux inverse 28 jusque dans la zone 30 au pied du stator. Cette zone 30 constitue un coin dans lequel le fluide, c'est-à-dire le sang dans le présent exemple, peut stagner. Le fait de générer un flux inverse de ce type en utilisant le mouvement de rotation de la turbine permet de maintenir un mouvement dans ce coin, donc d'éviter la stagnation du sang. On évite ainsi toute formation de thrombose.

La pale latérale 24 a une fonction de liaison entre l'arbre de transmission 20 et la cloche 21 et une fonction de guidage du sang vers le passage 29. Cette pale latérale 24 est reliée par une extrémité à une partie supérieure de la cloche 21, s'étend au-dessus de cette cloche, et est reliée par une autre extrémité à une partie basse de l'arbre de transmission 20. Ainsi disposée, la pale latérale 24 présente une partie centrale dans la chambre d'admission 16. Le passage 29 est l'entrefer entre le rotor et le stator et débouche au niveau de l'apex. Le passage 29 ne comporte pas de courbure prononcée afin d'éviter la création de zone de stagnation.

En rotation, l'arbre de transmission est en sustentation magnétique. L'extrémité inférieure de l'arbre de transmission comporte une zone arrondie convexe et constitue le pivot d'entrée 31. La partie supérieure du stator est en forme arrondie concave et constitue une zone de pivot 32. Le pivot d'entrée 31 et la zone de pivot 32 présentent des formes complémentaires qui peuvent s'épouser parfaitement. En position de repos, le pivot d'entrée 31 repose dans la zone de pivot 32. En rotation, l'arbre de transmission est en sustentation et un espacement se crée entre le pivot d'entrée 31 et la zone de pivot 32. Toutefois, la zone de pivot 32 est conformée de façon à maintenir le pivot d'entrée 31 dans son axe de rotation. La forme convexe du pivot d'entrée 31 permet en outre le passage du flux inverse dans l'espacement entre le pivot d'entrée 31 et la zone de pivot 32. Ce qui permet le nettoyage permanent de cette zone, donc peu de risque pour une stagnation sanguine.

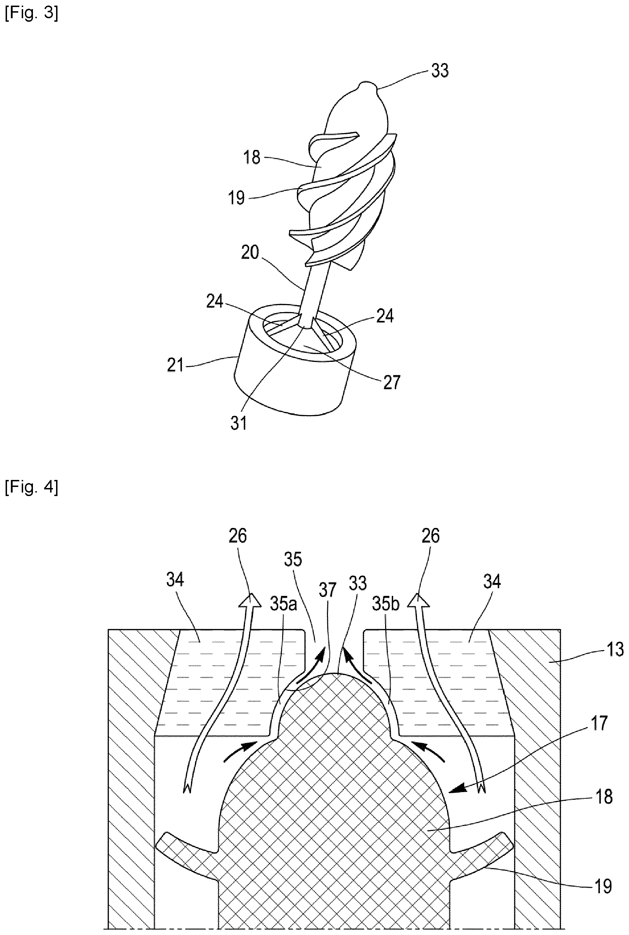

Sur la figure 3 est représentée un peu plus en détail la turbine 17 reliée à l'arbre de transmission 20 et à la cloche 21. Le pivot de sortie 33, en forme de demi-sphère, se trouve au sommet de la turbine et dans l'axe de rotation. Les pales hélicoïdales 19 entourent le corps 18 depuis le pied jusqu'à couvrir environ les trois-quarts du corps 18. Le sommet de la turbine est dépourvu de pales.

L'arbre de transmission 20 est solidement connecté à des pales latérales 24 dont deux sont visibles et une troisième cachée. Les pales latérales sont au nombre de trois, mais il peut y en avoir qu'une, deux ou plus que trois. Dans tous les cas, les pales latérales 24 doivent laisser des ouvertures 27 pour que le fluide puisse pénétrer à l'intérieur de la cloche 21. Dans l'exemple illustré sur les figures, le pivot d'entrée 31 se trouve au-dessus de la cloche 21, mais on peut envisager d'autres modes de réalisation où le pivot d'entrée est disposé à l'intérieur de la cloche 21.

Avec la pompe selon l'invention, l'arbre de transmission n'est pas mécaniquement connecté au stator du moteur.

Sur la figure 4 est représentée un peu plus en détail une partie supérieure de la pompe. On distingue la turbine 17 qui propulse le flux principal 26 vers la sortie de la pompe au moyen des pales 19. Le maintien en place de la turbine est assuré par la présence du pivot de sortie 33 de forme arrondie convexe. Il s'agit d'une demi-sphère. Ce pivot de sortie 33 est destinée à effectuer des mouvements de rotation, ensemble avec la turbine, tout en restant cantonnée dans une niche réalisée dans un guide fixe 34 reliée à la partie supérieure du carter 13. La niche est formée par des découpes 37 réalisées dans le guide 34. A forme de la niche est complémentaire à celle du pivot de sortie33. D'autres formes que celle de la demi-sphère peuvent être envisagées, telles que par exemple une forme conique. Cette niche constitue une zone de pivot pour le pivot de sortie33.

Les deux zones de pivot 32 et 37 permettent de maintenir l'ensemble turbine et arbre de transmission dans son axe de rotation et dans une zone de débattement verticale (selon le sens des schémas) assez limitée. De la même manière, pour éviter une stagnation du sang dans le guide 34, une ouverture 35 est réalisée au centre de ce guide de façon à permettre au sang de circuler et de ne plus stagner dans le guide. En effet, entre la zone 37, formant guide, et le pivot de sortie 33 se trouve un espacement 35a, 35b, ou un interstice pour le passage de fluide lorsque la pompe est en fonctionnement. Par l'action du moteur, on maintient un flux dans cet interstice de sorte que le sang ne puisse pas y stagner. On crée un passage 35a, 35b, depuis l'intérieur du carter jusque dans l'ouverture 35 via l'interstice.

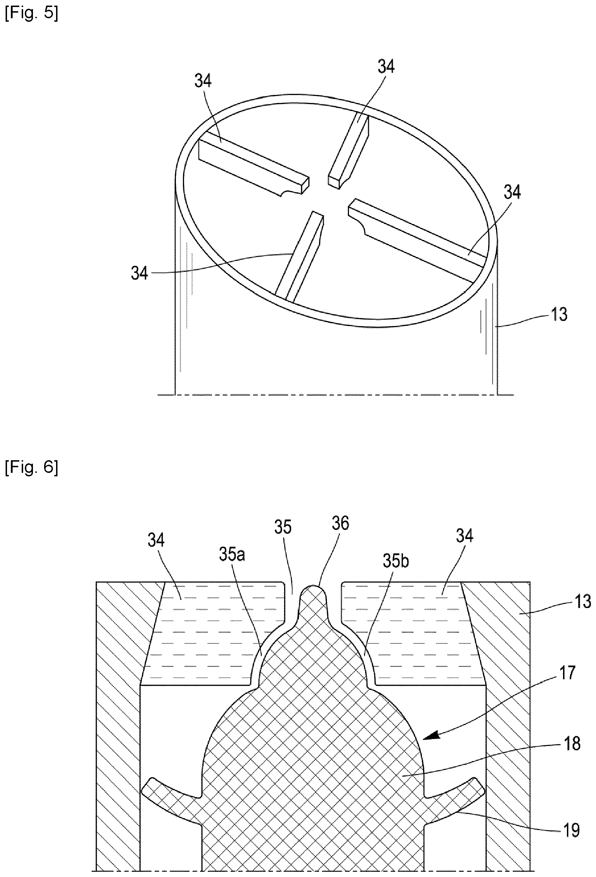

La figure 5 est une vue très schématique et simplifiée de la sortie de la pompe. On distingue des guides 34 qui sont des pales fixées à la paroi interne du carter 13. Il s'agit de quatre pales 34 disposées en croix et espacées les unes des autres dans la zone centrale de sorte que cette zone centrale constitue la niche et l'ouverture 35. L'ensemble des pales 34 constitue un redresseur disposé en sortie de la pompe de façon à augmenter la vitesse et donner au fluide un profil prédéterminé en sortie. Ce redresseur peut être précédé d'un diffuseur (« inducer » en anglais) de sortie non représenté sur la figure 4 et qui a pour fonction d'augmenter la pression du fluide de façon à évacuer le fluide depuis le rotor vers l'extérieur en transformant l'énergie cinétique créée par le rotor en énergie potentielle.

Avec une telle réalisation, le fluide passe principalement en tant que flux 26 comme on le voit sur la figure 4, mais il passe également via l'ouverture 35.

Pour une meilleure stabilité de la turbine, sur le mode de réalisation illustré sur la figure 6, on voit un pivot de sortie 33 dotée, dans son prolongement, d'une protubérance 36.

Cette protubérance 36 pénètre l'ouverture 35 partiellement ou complètement jusqu'à dépasser l'extrémité du guide. Le flux sanguin est propulsé alors le long de la paroi externe du pivot de sortie et de sa protubérance à travers l'ouverture 35 via les espacements 35a et 35b.

Avec la pompe selon l'invention, le pivot de sortie est contenu dans une niche à ouverture centrale. Cet arrangement permet d'éliminer toute zone de stagnation sanguine à l'origine d'éventuelles thromboses.

Le procédé préférentiel de fonctionnement de la pompe est un fonctionnement du moteur en mode pulsé.

Avec un tel fonctionnement, on prévoit des vitesses de 1500 à 7000 tours/mn.

Bien sûr, l'invention n'est pas limitée aux exemples qui viennent d'être décrits et de nombreux aménagements peuvent être apportés à ces exemples sans sortir du cadre de l'invention. Par exemple, les pivots peuvent être constitués l'un et ou l'autre d'un seul tenant avec la turbine ou l'arbre de transmission, mais ils peuvent également être l'un et/ou l'autre une pièce composée d'un matériau différent de celui de la turbine et de l'arbre de transmission, cette pièce serait fixée de manière permanente et serait en rotation avec la turbine et l'arbre de transmission. Ce matériau peut être du céramique ou une matière plastique thermostable comme du PEEK (polyétheréthercétone).

La zone de pivot sur le stator et/ou la partie 37 du guide peuvent être constituées d'un matériau en titane.

## Revendications

1. Pompe destinée à être immergée dans un fluide, comprenant :
- un carter (13, 14) fixe doté d'une partie supérieure (13) formant chambre de propulsion de fluide vers l'extrémité supérieure, et une partie inférieure (14) formant stator et reliée de manière fixe à la partie supérieure,
- au moins une ouverture latérale entre la partie supérieure et la partie inférieure, et formant une chambre d'admission (16) du fluide depuis l'extérieur vers la chambre de propulsion,
**caractérisée en ce que** la pompe comprend en outre un bloc moteur formé par :
- ledit stator (14) dans lequel sont disposés des premiers éléments magnétiques (22),
- un rotor (21) sous la forme d'une cloche coiffant au moins partiellement la tête du stator et comprenant des seconds éléments magnétiques destinés au couplage magnétique avec les premiers éléments magnétiques du stator, la cloche ayant au moins une ouverture (27) sur sa partie supérieure de façon à créer un flux inverse (28) du fluide depuis la chambre d'admission (16) jusqu'à la base du stator via un passage (29) entre le rotor et le stator,
- un arbre de transmission (20) comprenant au moins un bras de liaison (24) permettant de maintenir la cloche au-dessus du stator, l'axe de l'arbre de transmission étant superposé avec l'axe de rotation de la cloche.
- des pales latérales disposées entre la cloche (21) et l'arbre de transmission (20) et destinées à guider le flux inverse dans ledit passage(29) entre le rotor et le stator.

2. Pompe selon la revendication 1, **caractérisée en ce que** lesdites pales latérales sont orientées ou présentent une courbure.

3. Pompe selon la revendication 1 ou 2, **caractérisée en ce que** les bras de liaison (24) constituent lesdites pales latérales.

4. Pompe selon l'une quelconque des revendications précédentes, **caractérisée en ce qu'**elle est une pompe axiale.

5. Pompe selon l'une quelconque des revendications précédentes, **caractérisée en ce qu'**il s'agit d'une pompe cardiaque intraventriculaire destinée à être fixée à l'apex d'un cœur, le passage (29) débouchant directement dans la zone (30) de rencontre entre la paroi interne du ventricule et la paroi externe du stator.

6. Pompe selon la revendication 5, **caractérisée en ce qu'**elle présente des dimensions telles que la chambre d'admission se trouve à une distance de 1 à 3 cm de l'apex et l'orifice de sortie de la pompe se trouve à l'intérieur du ventricule à une distance de 1 à 3 cm en amont de la valve aortique.

7. Pompe selon l'une quelconque des revendications précédentes, **caractérisée en ce qu'**en fonctionnement la cloche et l'arbre de transmission sont destinés à être en sustentation magnétique grâce aux éléments magnétiques, l'extrémité inférieure (31) de l'arbre de transmission et l'extrémité supérieure (32) du stator coopérant de façon à maintenir l'arbre de transmission dans son axe de révolution en phase de rotation.

8. Pompe selon la revendication 7, **caractérisée en ce que** l'extrémité supérieure du stator comporte une zone de pivot concave apte à recevoir l'extrémité inférieure convexe, dite pivot d'entrée, de l'arbre de transmission.

9. Pompe selon la revendication 7, **caractérisée en ce que** l'extrémité supérieure (32) du stator comporte une zone de pivot convexe apte à recevoir l'extrémité inférieure (31) concave, dite pivot d'entrée, de l'arbre de transmission.

10. Pompe selon l'une quelconque des revendications précédentes, **caractérisée en ce que** l'arbre de transmission comporte une partie supérieure dotée de pales (19) permettant une aspiration de fluide principal depuis la chambre d'admission vers la sortie de la pompe.

11. Pompe selon la revendication 10, **caractérisée en ce qu'**elle comprend :
- à l'entrée de la partie supérieure (13) du carter, un inducteur doté de pales de guidage (25) pour rendre l'écoulement du fluide linéaire en direction de la partie supérieure de l'arbre de transmission ;
- la partie supérieure de l'arbre de transmission est un propulseur comprenant un corps central de forme évasée et destiné à créer de l'énergie cinétique;
- au moins une pale hélicoïdale (19) réalisée autour dudit corps central (18); cette pale hélicoïdale ayant un profil extérieur évasé et comportant des spires à pas d'enroulement croissant et tendant vers l'infini ; le volume intérieur du carter étant complémentaire à la forme évasée de ladite au moins une pale hélicoïdale.

12. Pompe selon l'une quelconque des revendications 1 à 9, **caractérisée en ce que** la partie supérieure de l'arbre de transmission est un propulseur comprenant une tige dans l'axe centrale fixée à une turbine conçue pour effectuer des mouvements de rotation relativement au carter, cette turbine étant un cylindre creux d'axe de révolution confondu avec ladite tige et destiné au passage du flux principal de fluide, cette turbine comprenant au moins une pale interne disposée sur sa paroi interne et conçue pour faire circuler le fluide provenant de la chambre d'admission.

13. Pompe selon l'une quelconque des revendications précédentes, **caractérisée en ce que** la partie supérieure de l'arbre de transmission est un propulseur comprenant un corps (18) destiné à créer de l'énergie cinétique, ce corps comprenant à son sommet un pivot de sortie apte à coopérer avec un guide (37, 34) fixé au carter (13), ce guide présentant une forme complémentaire à celle du pivot de sortie de façon à maintenir ce pivot de sortie stable en rotation et à former un espacement (35a, 35b) entre le pivot de sortie et le guide, et **en ce que** ce guide comprend une ouverture centrale (35) dans l'axe de révolution du pivot de sortie pour un passage (35a, 35b) de fluide depuis l'intérieur du carter vers l'extérieur via l'espacement et l'ouverture.

14. Pompe selon la revendication 13, **caractérisée en ce que** le pivot de sortie présente une forme convexe et le guide comprend une ouverture centrale (35) dans l'axe de révolution du pivot de sortie pour le passage de fluide depuis l'intérieur du carter vers l'extérieur.

15. Pompe selon la revendication 14, **caractérisée en ce que** le pivot de sortie comporte une protubérance (36) pénétrant dans l'ouverture du guide.

16. Pompe selon l'une quelconque des revendications 13 à 15, **caractérisée en ce que** le guide est constitué d'au moins une pale d'un redresseur et/ou d'un diffuseur.

17. Pompe selon l'une quelconque des revendications précédentes, **caractérisée en ce qu'**il s'agit d'une pompe cardiaque intraventriculaire destinée à être fixée à l'apex d'un ventricule gauche ou d'un ventricule droit ou d'un ventricule systémique.

## Patentansprüche

1. Pumpe, die dazu vorgesehen ist, in ein Fluid eingetaucht zu werden, umfassend:
- ein festes Gehäuse (13, 14) mit einem oberen Teil (13), der eine Kammer zum Antreiben von Fluid zum oberen Ende hin bildet, und einem unteren Teil (14), der einen Stator bildet und fest mit dem oberen Teil verbunden ist,
- mindestens eine seitliche Öffnung zwischen dem oberen Teil und dem unteren Teil, die eine Einlasskammer (16) für das Fluid von außen in die Antriebskammer bildet, **dadurch gekennzeichnet, dass** die Pumpe ferner einen Motorblock umfasst, der gebildet ist durch:
- den genannten Stator (14), in dem erste magnetische Elemente (22) angeordnet sind,
- einen Rotor (21) in Form einer Glocke, die den Kopf des Stators zumindest teilweise überdeckt und zweite magnetische Elemente umfasst, die zur magnetischen Kopplung mit den ersten magnetischen Elementen des Stators vorgesehen sind, wobei die Glocke mindestens eine Öffnung (27) an ihrem oberen Teil aufweist, so dass eine Rückströmung (28) des Fluids von der Einlasskammer (16) bis zur Basis des Stators über eine Passage (29) zwischen dem Rotor und dem Stator erzeugt wird,
- eine Antriebswelle (20) umfassend mindestens einen Verbindungsarm (24), mit dem die Glocke über dem Stator gehalten werden kann, wobei die Achse der Antriebswelle mit der Drehachse der Glocke zusammenfällt,
- seitliche Schaufeln, die zwischen der Glocke (21) und der Antriebswelle (20) angeordnet sind und dazu vorgesehen sind, die Rückströmung in der Passage (29) zwischen dem Rotor und dem Stator zu führen.

2. Pumpe nach Anspruch 1, **dadurch gekennzeichnet, dass** die genannten seitlichen Schaufeln orientiert sind oder eine Krümmung aufweisen.

3. Pumpe nach Anspruch 1 oder 2, **dadurch gekennzeichnet, dass** die Verbindungsarme (24) die seitlichen Schaufeln ausbilden.

4. Pumpe nach einem der vorhergehenden Ansprüche, **dadurch gekennzeichnet, dass** diese eine Axialpumpe ist.

5. Pumpe nach einem der vorhergehenden Ansprüche, **dadurch gekennzeichnet, dass** es sich um eine intraventrikuläre Herzpumpe zur Anordnung an der Spitze eines Herzens handelt, wobei die Passage (29) direkt in den Bereich (30) des Zusammentreffens zwischen der Innenwand des Ventrikels und der Außenwand des Stators mündet.

6. Pumpe nach Anspruch 5, **dadurch gekennzeichnet, dass** diese so dimensioniert ist, dass sich die Einlasskammer in einem Abstand von 1 bis 3 cm von der Spitze befindet und sich die Ausgangsöffnung der Pumpe im Inneren des Ventrikels in einem Abstand von 1 bis 3 cm stromaufwärts des Aortenventils befindet.

7. Pumpe nach einem der vorhergehenden Ansprüche, **dadurch gekennzeichnet, dass** im Betrieb die Glocke und die Antriebswelle dazu vorgesehen sind, mithilfe der magnetischen Elemente in einem magnetischen Schwebezustand zu sein, wobei das untere Ende (31) der Antriebswelle und das obere Ende (32) des Stators derart zusammenwirken, dass die Antriebswelle in der Rotationsphase in ihrer Umdrehungsachse gehalten wird.

8. Pumpe nach Anspruch 7, **dadurch gekennzeichnet, dass** das obere Ende des Stators einen konkaven Drehzapfenbereich aufweist, der dazu geeignet ist, das untere konvexe Ende, auch Eingangsdrehzapfen genannt, der Antriebswelle aufzunehmen.

9. Pumpe nach Anspruch 7, **dadurch gekennzeichnet, dass** das obere Ende (32) des Stators einen konvexen Drehzapfenbereich aufweist, der dazu geeignet ist, das konkave untere Ende (31), auch Eingangsdrehzapfen genannt, der Antriebswelle aufzunehmen.

10. Pumpe nach einem der vorhergehenden Ansprüche, **dadurch gekennzeichnet, dass** die Antriebswelle einen oberen Teil mit Schaufeln (19) aufweist, die ein Ansaugen des Hauptfluids von der Einlasskammer zum Ausgang der Pumpe ermöglichen.

11. Pumpe nach Anspruch 10, **dadurch gekennzeichnet, dass** diese umfasst:
- am Eingang des oberen Teils (13) des Gehäuses, einen Induktor mit Leitschaufeln (25), um den Fluidströmungsverlauf geradlinig in Richtung des oberen Teils der Antriebswelle werden zu lassen;
- wobei der obere Teil der Antriebswelle ein Triebwerk ist, das einen zentralen Körper mit einer aufgeweiteten Form aufweist und zur Erzeugung von kinetischer Energie vorgesehen ist;
- mindestens eine um den zentralen Körper (18) herum ausgebildete wendeiförmige Schaufel (19), die ein aufgeweitetes Außenprofil besitzt und Windungen mit zunehmender und gegen unendlich strebender Steigung aufweist, wobei das Innenvolumen des Gehäuses komplementär zu der aufgeweiteten Form der mindestens einen wendeiförmigen Schaufel ist.

12. Pumpe nach einem der Ansprüche 1 bis 9, **dadurch gekennzeichnet, dass** der obere Teil der Antriebswelle ein Triebwerk mit einem Schaft in der Mittelachse ist, der an einer zur Ausführung von Rotationsbewegungen relativ zum Gehäuse ausgelegten Turbine gelagert ist, wobei diese Turbine ein Hohlzylinder mit einer mit dem genannten Schaft zusammenfallenden Umdrehungsachse ist und für den Durchgang der Hauptströmung des Fluids vorgesehen ist, wobei diese Turbine mindestens eine innere Schaufel aufweist, die an ihrer Innenwand angeordnet und dazu vorgesehen ist, das aus der Einlasskammer kommende Fluid zum Zirkulieren zu bringen.

13. Pumpe nach einem der vorhergehenden Ansprüche, **dadurch gekennzeichnet, dass** der obere Teil der Antriebswelle ein Triebwerk ist, das einen zur Erzeugung von kinetischer Energie vorgesehenen Körper (18) umfasst, wobei dieser Körper an seiner Spitze einen Ausgangsdrehzapfen umfasst, der dazu geeignet ist, mit einer am Gehäuse (13) angeordneten Führung (37, 34) zusammenzuwirken, wobei diese Führung eine Form aufweist, die komplementär zu derjenigen des Ausgangsdrehzapfens ist, um diesen Ausgangsdrehzapfen in Drehung stabil zu halten und einen Zwischenraum (35a, 35b) zwischen dem Ausgangsdrehzapfen und der Führung zu bilden, und dass diese Führung eine zentrale Öffnung (35) an der Umdrehungsachse des Ausgangsdrehzapfens für einen Durchgang (35a, 35b) von Fluid aus dem Inneren des Gehäuses nach außen über den Zwischenraum und die Öffnung aufweist.

14. Pumpe nach Anspruch 13, **dadurch gekennzeichnet, dass** der Ausgangsdrehzapfen eine konvexe Form aufweist und die Führung eine zentrale Öffnung (35) an der Umdrehungsachse des Ausgangsdrehzapfens für den Durchgang von Fluid aus dem Inneren des Gehäuses nach außen aufweist.

15. Pumpe nach Anspruch 14, **dadurch gekennzeichnet, dass** der Ausgangsdrehzapfen einen in die Öffnung der Führung hineinreichenden Vorsprung (36) aufweist.

16. Pumpe nach einem der Ansprüche 13 bis 15, **dadurch gekennzeichnet, dass** die Führung aus mindestens einer Schaufel eines Strömungsrichters und/oder eines Diffusors gebildet ist.

17. Pumpe nach einem der vorhergehenden Ansprüche, **dadurch gekennzeichnet, dass** es sich um eine intraventrikuläre Herzpumpe handelt, die zur Anordnung an der Spitze eines linken Ventrikels oder eines rechten Ventrikels oder eines systemischen Ventrikels vorgesehen ist.

## Claims

1. Pump intended to be immersed in a fluid, comprising:
- a fixed housing (13, 14) provided with a top part (13) forming a propulsion chamber propelling fluid towards the top end, and a bottom part (14) forming a stator and attached in a fixed manner to the top part,
- at least one side opening between the top part and the bottom part, and forming an inlet chamber (16) for the fluid to enter from the exterior towards the propulsion chamber,
**characterized in that** the pump also comprises a motor unit formed by:
- said stator (14) in which first magnetic elements (22) are arranged,
- a rotor (21) in the form of a bell at least partially covering the stator head and comprising second magnetic elements intended for magnetic coupling with the first magnetic elements of the stator, the bell having at least one opening (27) in its top part so as to create a reverse flow (28) of the fluid from the inlet chamber (16) right up to the base of the stator via a passage (29) between the rotor and the stator,
- a transmission shaft (20) comprising at least one connecting arm (24) making it possible to hold the bell above the stator, the axis of the transmission shaft being superposed with the axis of rotation of the bell
- side vanes arranged between the bell (21) and the transmission shaft (20) and intended to guide the reverse flow in said passage (29) between the rotor and the stator.

2. Pump according to claim 1, **characterized in that** said side vanes are directional or have a curvature.

3. Pump according to claim 1 or 2, **characterized in that** the connecting arms (24) constitute said side vanes.

4. Pump according to any one of the preceding claims, **characterized in that** it is an axial pump.

5. Pump according to any one of the preceding claims, **characterized in that** it is an intraventricular heart pump intended to be fixed at the apex of a heart, the passage (29) opening directly in the zone (30) where the internal wall of the ventricle and the external wall of the stator meet.

6. Pump according to claim 5, **characterized in that** it has dimensions such that the inlet chamber is located at a distance of 1 to 3 cm from the apex and the outlet orifice of the pump is located in the interior of the ventricle at a distance of 1 to 3 cm upstream of the aortic valve.

7. Pump according to any one of the preceding claims, **characterized in that** in operation the bell and the transmission shaft are intended to be in magnetic suspension by virtue of the magnetic elements, the bottom end (31) of the transmission shaft and the top end (32) of the stator cooperating so as to hold the transmission shaft within its axis of revolution in the rotation phase.

8. Pump according to claim 7, **characterized in that** the top end of the stator includes a concave pivot zone suitable for receiving the convex bottom end, called inlet pivot, of the transmission shaft.

9. Pump according to claim 7, **characterized in that** the top end (32) of the stator includes a convex pivot zone suitable for receiving the concave bottom end (31), called inlet pivot, of the transmission shaft.

10. Pump according to any one of the preceding claims, **characterized in that** the transmission shaft includes a top part equipped with vanes (19), allowing main fluid to be drawn from the inlet chamber to the outlet of the pump.

11. Pump according to claim 10, **characterized in that** it comprises:
- at the inlet of the top part (13) of the housing, an inductor equipped with guide vanes (25) in order to make the flow of the fluid linear in the direction of the top part of the transmission shaft;
- the top part of the transmission shaft is a propeller comprising a central body with a flared shape, intended to create kinetic energy;
- at least one helical vane (19), produced around said central body (18); this helical vane having a flared external profile and including turns having an increasing winding pitch that tends towards infinity; the internal volume of the housing being complementary to the flared shape of said at least one helical vane.

12. Pump according to any one of claims 1 to 9, **characterized in that** the top part of the transmission shaft is a propeller comprising a rod within the central axis fixed to an impeller designed to perform rotational movements with respect to the housing, this impeller being a hollow cylinder with axis of revolution merged with said rod and intended for the main fluid flow to pass, this impeller comprising at least one internal vane arranged on its internal wall and designed to circulate the fluid originating from the inlet chamber.

13. Pump according to any one of the preceding claims, **characterized in that** the top part of the transmission shaft is a propeller comprising a body (18) intended to create kinetic energy, this body comprising at its apex an outlet pivot suitable for cooperating with a guide (37, 34) fixed to the housing (13), this guide having a shape complementary to that of the outlet pivot so as to hold this outlet pivot stable in rotation and to form a clearance (35a, 35b) between the outlet pivot and the guide, and **in that** this guide comprises a central opening (35) within the axis of revolution of the outlet pivot for a passage (35a, 35b) for fluid from the interior of the housing to the exterior, via the clearance and the opening.

14. Pump according to claim 13, **characterized in that** the outlet pivot has a convex shape and the guide comprises a central opening (35) within the axis of revolution of the outlet pivot for fluid to pass from the interior of the housing to the exterior.

15. Pump according to claim 14, **characterized in that** the outlet pivot includes a projection (36) entering the opening of the guide.

16. Pump according to any one of claims 13 to 15, **characterized in that** the guide is constituted by at least one vane of a straightener and/or an inducer.

17. Pump according to any one of the preceding claims, **characterized in that** it is an intraventricular heart pump intended to be fixed at the apex of a left ventricle or of a right ventricle or of a systemic ventricle.
